# EUROPÄISCHE PATENTSCHRIFT

(11) **EP 2 222 997 B1**
(45) Veröffentlichungstag und Bekanntmachung des Hinweises auf die Patenterteilung: **04.05.2011**
(21) Anmeldenummer: 08862932.4
(22) Anmeldetag: 17.11.2008
(51) Int. Cl.: F16L 37/02, F16L 37/084, A61M 16/08

(54) **ALS RADIALLÜFTER AUSGEBILDETER KLEIN- ODER KLEINSTLÜFTER**
MINIATURE FAN OR MICRO-FAN DESIGNED AS A RADIAL FAN, RESPECTIVELY
VENTILATEUR MINIATURE OU MICRO-VENTILATEUR SOUS FORME D'UN VENTILATEUR RADIAL, RESPECTIVEMENT

(30) Priorität: 16.12.2007 DE 202007018175 U
(43) Veröffentlichungstag der Anmeldung: 01.09.2010
(73) Patentinhaber: Ebm-Papst St. Georgen GmbH & CO. KG, 78112 St. Georgen (DE)
(72) Erfinder: KUHNERT, Gerhard, 78098 Unterkirnach (DE)
(74) Vertreter: Raible, Hans
(86) Internationale Anmeldenummer: PCT/EP2008/009703
(87) Internationale Veröffentlichungsnummer: WO 2009/077045

(56) Entgegenhaltungen:
- EP-A- 0 237 904
- EP-A- 0 872 643
- WO-A-2006/015772
- WO-A-2006/133480
- DE-C- 404 941
- DE-U1- 20 016 769
- US-A- 367 578
- US-A- 3 413 021

## Beschreibung

Die Erfindung betrifft einen als Radiallüfter ausgebildeten Klein- oder Kleinstlüfter.

Für viele Anwendungen werden heute sog. Kleinlüfter verwendet, z. B. in Beatmungsgeräten für Patienten. Solche Kleinlüfter, in vielen Fällen Radiallüfter, haben einen Anschlussstutzen für die ausgeblasene Luft, und dieser Stutzen muss mit einem geeigneten Rohr so verbunden werden, dass die Verbindung mit dem Anschlussstutzen im Rahmen der Massenfertigung einfach und schnell, dabei aber prozesssicher, hergestellt werden kann. Als Rohr verwendet man häufig ein Rohr, das im sog. Blasverfahren oder im Extrusionsblasverfahren hergestellt wird und das deshalb - sprachlich verkürzt - auch als "Blasrohr" bezeichnet wird.

**Zum Anschluss der Gebläse von Beatmungsgeräten kennt man Steckverbindungen, vgl.** EP 0 872 643 A2
DE 200 16 769 U1

Aus dem Stand der Technik sind Steckverbindungen für Rohre bekannt, z. B. aus
DE 404 941
US 367 578
US 3 413 021
WO 2006/015 772 A1
WO 2006/133480 A1
EP 0 237 904 A1.

Nachteilig bei diesen Steckverbindungen ist, dass sie bei ihrer Anordnung an einem Gerät dessen Abmessungen vergrößern und dadurch das Gerät verteuern.

Deshalb ist es eine Aufgabe der Erfindung, einen neuen Lüfter bereit zu stellen, der ohne wesentliche Zusatzkosten verbesserte Eigenschaften hat.

Nach der Erfindung wird diese Aufgabe gelöst durch den Gegenstand des Anspruchs 1.

Da bei der Erfindung eine Trennfuge verwendet wird, die bei einem Radiallüfter ohnedies vorhanden ist, kann die Erfindung praktisch ohne Mehrkosten realisiert werden, da hierbei lediglich die Trennfuge im Bereich des Auslassstutzens etwas verbreitert werden muss und sich keine Änderungen der Abmessungen eines solchen Lüfters ergeben.

Man erreicht so, dass die Verbindung eines Rohrendabschnitts mit dem Anschlussstutzen eines Lüfters schnell und prozesssicher möglich ist, auch bei Verhältnissen, wo die Verbindung "blind", also ohne Sichtkontakt, hergestellt werden muss, denn man erhält beim Einrasten ein entsprechendes Geräusch als akustische Quittung dafür, dass die Verbindung ordnungsgemäß hergestellt wurde.

Durch den Gegenstand des Anspruchs 2 erhält man zusätzlich eine Verringerung der luftverluste an der Anschlussstelle, was besonders bei batteriebetriebenen Geräten wichtig ist, da man dann oft nur einen sehr kleinen Lüfter braucht, was den Stromverbrauch niedrig hält. Dies ist z.B. wichtig bei batteriebetriebenen Geräten, die bei intermittierender Atmung benötigt werden, um die Sauerstoffversorgung eines Patienten sicher zu stellen, z.B. nach einem Schädel-Hirn-Trauma.

Weitere Einzelheiten und vorteilhafte Weiterbildungen der Erfindung ergeben sich aus den in der Zeichnung dargestellten und im folgenden beschriebenen Ausführungsbeispielen, sowie aus den Unteransprüchen. Es zeigt:
- Fig. 1: eine raumbildliche Darstellung eines Kleinlüfters mit einem Anschlussstutzen 22 auf dessen Ausblasseite, an welchen Stutzen ein Blasrohr 52 angeschlossen werden soll, das in Fig. 1, oben, dargestellt ist,
- Fig. 2: eine Darstellung analog Fig. 1, bei der der Rohrendabschnitt des Blasrohrs in dem Zustand vor dessen Anschluss im Längsschnitt dargestellt ist,
- Fig. 3: eine Darstellung analog Fig. 2, bei welcher der Rohrendabschnitt bereits teilweise auf den Anschlussstutzen des Kleinlüfters aufgeschoben ist, und
- Fig. 4: eine Darstellung analog Fig. 2 und 3, aber in fertig montiertem Zustand.

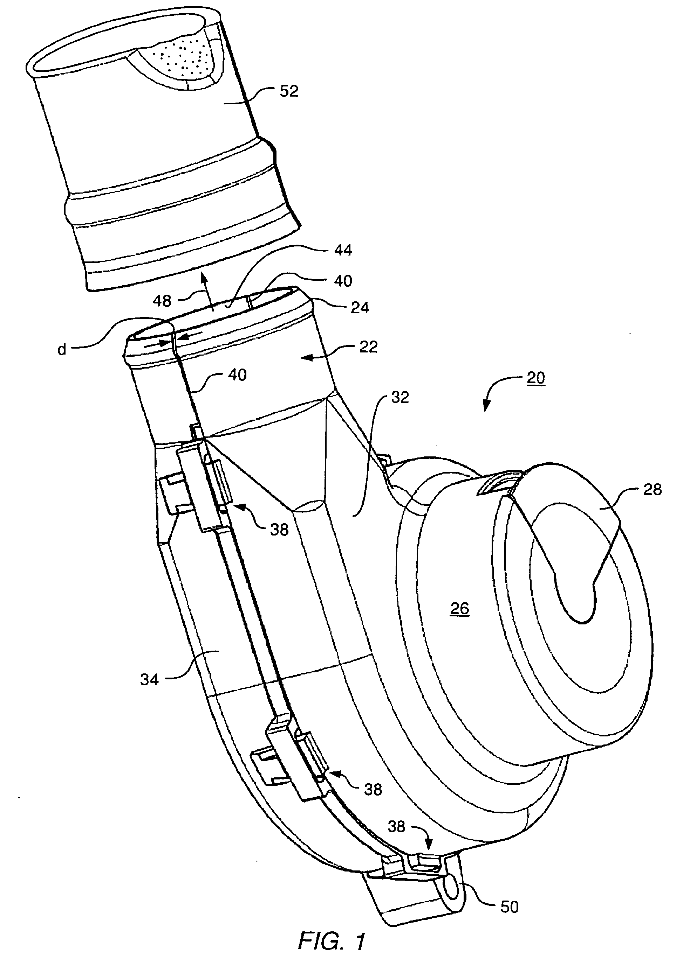

Fig. 1 zeigt, etwa zweifach vergrößert, einen Kleinlüfter 20, der auf seiner Ausblasseite einen Anschlussstutzen 22 hat, an dem sich ein Rastwulst 24 befindet.

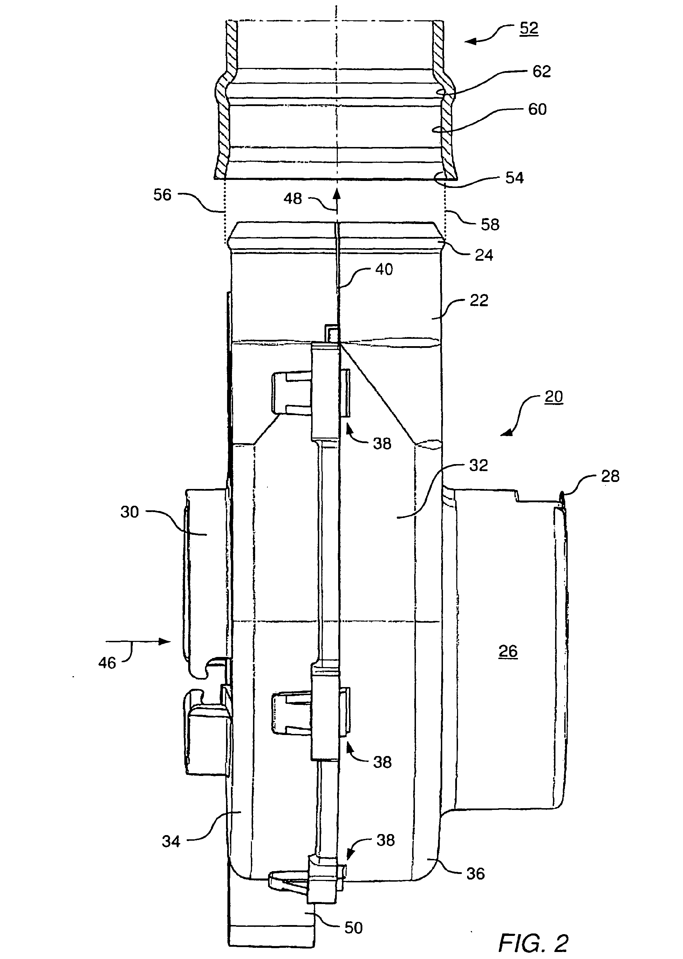

Der dargestellte Lüfter 20 ist ein modifizierter Radiallüfter vom Typ RV40 aus dem Programm der Anmelderin. Dieser hat rechts einen elektronisch kommutierten Außenläufermotor 26 mit einem aufklappbaren Deckel, unter dem, nach dem Aufklappen, nicht dargestellte elektrische Leitungen angeschlossen werden können. Auf der dem Motor 26 gegenüber liegenden Seite befindet sich eine Ansaugöffnung 30 (Fig. 2), durch welche Luft in das (nicht dargestellte) Radiallüfterrad des Lüfters 20 angesaugt und dann durch dieses in einem Spiralgehäuse 32 verdichtet und dem Stutzen 22 auf der Ausblasseite zugeführt wird. Die Strömungsrichtung der Luft ist durch Pfeile 46 (Saugseite) und 48 (Ausblasseite) dargestellt.

Das Spiralgehäuse 32 ist aus glasfaserverstärktem halogenfreiem Kunststoff hergestellt und daher elastisch. Es hat ein saugseitiges Teil 34 und ein motorseitiges Teil 36. Die Teile 34, 36 sind durch Verbindungselemente 38 von bekannter Bauart, von denen nur ein Teil dargestellt ist, und durch eine überlappte Nut-Rillen-Verbindung, luftdicht zusammengehalten und haben deshalb eine Trennfuge 40, die im Bereich des Anschlussstutzens 22 offen liegt, (also nicht überlappt) und sich in Richtung zum Auslassende 44 verbreitert. Je nach Anwendung können mehrere solche Trennfugen verwendet werden. Beim dargestellten Beispiel, wo der Stutzen 22 an seinem Wulst 24 einen Außendurchmesser von z.B. 31 mm hat, beträgt die Breite d der Trennfugen 40 am freien Ende des Anschlussstutzens 22 je etwa 0,5 bis etwa 0,8 mm und nimmt in Richtung zum Spiralgehäuse 32 in der dargestellten Weise auf den Wert Null ab. Dadurch ist der Anschlussstutzen 22 des modifizierten Lüfters 20 im Bereich seines freien Endes radial federnd, was das Zusammenstecken erleichtert und die Dichtigkeit der Steckverbindung verbessert.

Zur Montage ist am Gehäuseteil 34 ein erstes Montageauge 50 vorgesehen. Ein zweites Montageauge ist in den Fig. 1 bis 4 nicht sichtbar. -- Die Breite d hängt u.a. vom Durchmesser des Ausblasstutzens 22 ab, nimmt also mit zunehmendem Durchmesser des Stutzens 22 zu.

Am Stutzen 22 wird ein Rohr 52 befestigt. Die Montage wird anhand der Fig. 2 bis 4 erläutert.

Fig. 2 zeigt den Radiallüfter 20, an den ein Blasrohr 52 angekoppelt werden soll, dessen Rohrendabschnitt im Längsschnitt dargestellt ist und dessen Länge, wie aus Fig. 1 hervorgeht, entsprechend den Bedürfnissen variiert.

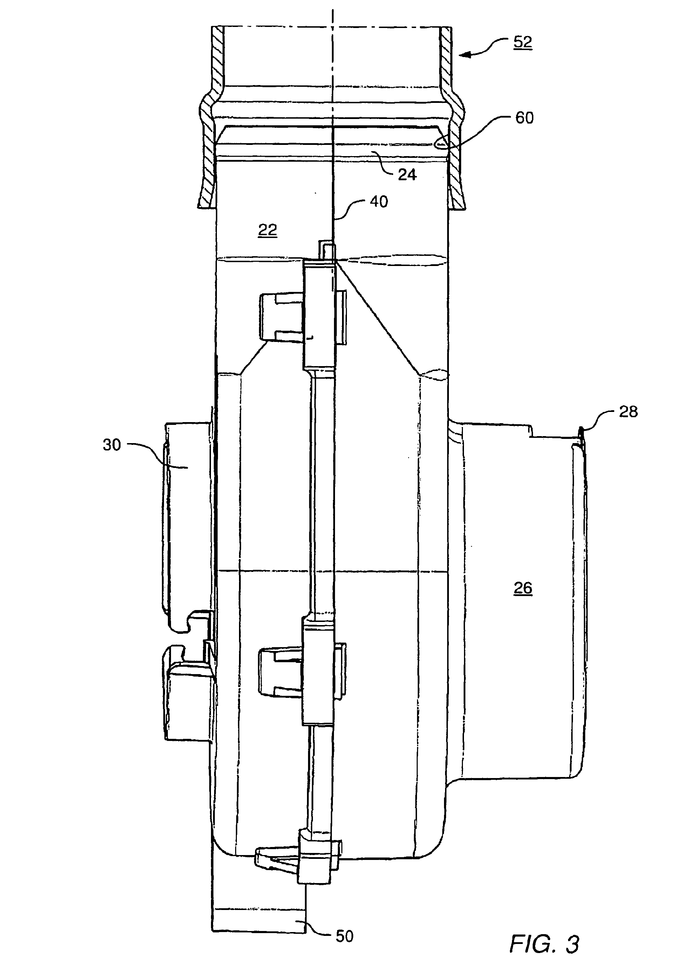

Das Rohr 52 hat an seinem dem Lüfter 20 zugewandten freien Ende eine etwa trichterförmige Öffnung. Wie die Linien 56, 58 zeigen, ist der größte Durchmesser des Trichters 54 etwa so gewählt, dass bei der Montage dieser Trichter 54 einfach auf den Rastwulst 24 aufgesetzt werden kann, um dort den Längsspalt 40 zusammen zu pressen und dadurch ein Einführen des Rastwulstes 24 in einen hohlzylindrischen Abschnitt 60 des Blasrohres 52 zu ermöglichen, wie das in Fig. 3 dargestellt ist.

Vergleicht man Fig. 2 mit Fig. 3, so sind in Fig. 2 die Längsspalte 40 geöffnet, und in Fig. 3 sind sie zusammengepresst, so dass der Rastwulst 24 mit radialer Vorspannung gegen die Innenseite des Abschnitts 60 anliegt.

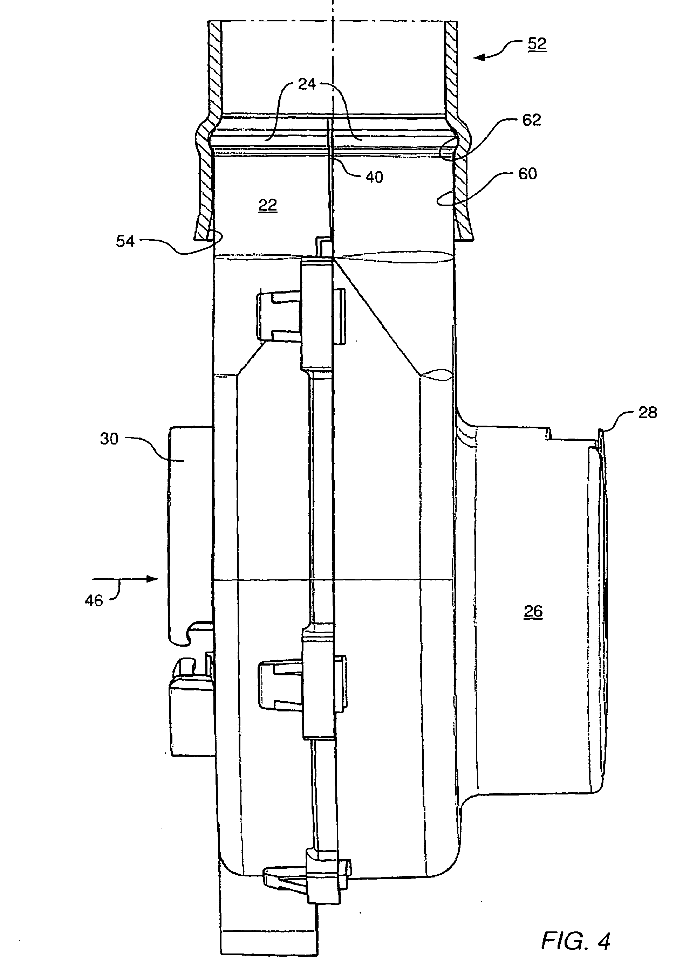

Wird nun gemäß Fig. 4 das Rohr 52 noch weiter auf den Stutzen 22 aufgeschoben, so rastet der Rastwulst 24 federnd in eine zu ihm etwa komplementäre Rastnut 62 ein, deren Form am besten aus Fig. 2 hervorgeht. Dabei weitet sich der Längsspalt 40 wieder auf, wie in Fig. 4 dargestellt, so dass der Rastwulst 24 hörbar in die Rastnut 62 einrastet und die Montageperson eine entsprechende hörbare Quittung erhält.

Gleichzeitig wird der Außenumfang des Luftaustrittsstutzens 22 federnd gegen den zylindrischen Abschnitt 60 des Rohrendabschnitts gepresst, so dass hierdurch der Spalt 40 bzw. die Spalte 40 zusätzlich verschlossen wird bzw. werden.

Die Demontage geht in umgekehrter Richtung in gleicher Weise vor sich, d.h. der Rohrendabschnitt 52 kann in einfacher Weise vom Stutzen 22 abgezogen werden. Die Verbindung ist also preiswert, praxisgerecht, und sehr prozesssicher.

## Patentansprüche

1. Als Radiallüfter ausgebildeter Klein- oder Kleinstlüfter,
mit einem Spiralgehäuse (32), das mit einem Luftaustrittsstutzen (22) verbunden ist, durch den im Betrieb die Luft aus dem Radiallüfter (20) austritt,
mit einer Trennfuge (40) zwischen Gehäuseteilen (34, 36) des Radiallüfters, welche Trennfuge (40) sich im nicht zusammengepressten Zustand im Bereich des Luftaustrittsstutzens (22) in Richtung zum freien Ende des Luftaustrittsstutzens erweitert und einen Spalt (40) bildet,
welcher Luftaustrittsstutzen (22) zur Verbindung mit einem auf seiner Innenseite mit einer Rastnut (62) versehenen Rohrendabschnitt (52) mit einem Rastwulst (24) ausgebildet ist, durch welchen sich die Trennfuge (40) erstreckt, so dass der Luftaustrittsstutzen (22) beim Einführen in einen solchen Rohrendabschnitt (52) federnd zusammenpressbar ist, um eine schnelle Verbindung mit einem solchen Rohrendabschnitt (52) herstellen zu können.

2. Lüfter nach Anspruch 1, bei welchem der Luftaustrittsstutzen (22) so ausgebildet ist, dass er nach dem Einrasten seines Rastwulstes in die Rastnut (62) eines Rohrendabschnitts (52) im elastisch aufgefederten Zustand mit geringem oder keinem Abstand gegen die Innenseite des Rohrendabschnitts (52) anliegt.

3. Lüfter nach Anspruch 1 oder 2, bei welchem im Luftaustrittsstutzen (22) eine Mehrzahl von Schlitzen (40) vorgesehen ist, um den Luftaustrittsstutzen (22) federnd zusammenpressbar zu machen.

4. Lüfter nach einem der vorhergehenden Ansprüche, bei welchem der mindestens eine Schlitz (40) seine größte Breite (d) im Bereich der Austrittsöffnung des Luftaustrittsstutzens (22) hat.

5. Lüfter nach Anspruch 4, bei welchem die Breite (d) des mindestens einen Schlitzes (40) in Richtung weg von der Austrittsöffnung des Luftaustrittsstutzens (22) abnimmt.

## Claims

1. A miniature or micro-fan formed as a radial fan,
with a spiral housing (32) which is connected to an air exit port (22) through which the air emerges from the radial fan (20) during operation,
with a parting line (40) between housing parts (34, 36) of the radial fan, which parting line (40), in the non-pressed-together state, in the region of the air exit port (22) widens in the direction of the free end of the air exit port and forms a gap (40),
which air exit port (22), for connection to a tube end section (52) which is provided on its inside with a latch groove (62), is formed with a latch bead (24) through which the parting line (40) extends, so that the air exit port (22), upon introduction into such a tube end section (52), can be pressed together in resilient manner in order to be able to bring about a rapid connection to such a tube end section (52).

2. A fan according to Claim 1, in which the air exit port (22) is formed such that, once its latch bead has engaged in the latch groove (62) of a tube end section (52), it lies in the elastically sprung-apart state with a slight or no distance against the inside of the tube end section (52).

3. A fan according to Claim 1 or 2, in which a plurality of slits (40) is provided in the air exit port (22) in order to make the air exit port (22) able to be pressed resiliently together.

4. A fan according to one of the preceding claims, in which the at least one slit (40) has its greatest width (d) in the region of the exit opening of the air exit port (22).

5. A fan according to Claim 4, in which the width (d) of the at least one slit (40) decreases in the direction away from the exit opening of the air exit port (22).

## Revendications

1. Ventilateur miniature ou microventilateur réalisé sous la forme d'un ventilateur radial, comprenant
un carter spiroïdal (32) relié à un manchon (22) de sortie d'air par lequel l'air sort, en service, dudit ventilateur radial (20),
un joint de séparation (40) situé entre des parties (34, 36) du carter dudit ventilateur radial, lequel joint de séparation (40) s'évase dans la région du manchon (22) de sortie d'air à l'état non comprimé, en direction de l'extrémité libre dudit manchon de sortie d'air, et forme un interstice (40),
lequel manchon (22) de sortie d'air est muni, en vue de la liaison avec un tronçon tubulaire extrême (52) présentant une rainure d'encliquetage (62) sur sa face intérieure, d'un bourrelet encliquetable (24) par lequel le joint de séparation (40) s'étend, de telle sorte que ledit manchon (22) de sortie d'air puisse être comprimé élastiquement lors de l'insertion dans un tel tronçon tubulaire extrême (52), afin de pouvoir instaurer une liaison rapide avec un tel tronçon tubulaire extrême (52).

2. Ventilateur selon la revendication 1, dans lequel le manchon (22) de sortie d'air est réalisé de façon telle qu'à l'issue du crantage de son bourrelet encliquetable dans la rainure d'encliquetage (62) d'un tronçon tubulaire extrême (52), il porte contre la face intérieure dudit tronçon tubulaire extrême (52) à l'état de détente élastique, avec espacement faible ou nul.

3. Ventilateur selon la revendication 1 ou 2, dans lequel une pluralité de fentes (40) est prévue, dans le manchon (22) de sortie d'air, pour conférer une propriété de compressibilité élastique audit manchon (22) de sortie d'air.

4. Ventilateur selon l'une des revendications précédentes, dans lequel la fente (40), à présence minimale, offre sa largeur maximale (d) dans la région de l'orifice de sortie du manchon (22) de sortie d'air.

5. Ventilateur selon la revendication 4, dans lequel la largeur (d) de la fente (40) à présence minimale décroît dans une direction l'éloignant de l'orifice de sortie du manchon (22) de sortie d'air.
